(19) Europäisches Patentamt
European Patent Office
Office européen des brevets

(11) Publication number: 0 208 417
A2

# (12) EUROPEAN PATENT APPLICATION

(21) Application number: 86304362.6

(51) Int. Cl.4: A61K 31/48

(22) Date of filing: 09.06.86

The title of the invention has been amended (Guidelines for Examination in the EPO, A-III, 7.3).

(30) Priority: 12.06.85 CS 4246/85

(43) Date of publication of application: 14.01.87 Bulletin 87/03

(84) Designated Contracting States: BE CH DE FR GB IT LI LU NL SE

(71) Applicant: SPOFA spojené podniky pro zdravotnickou vyrobu
No 11a Husinecká
Praha 3(CS)

(72) Inventor: Crny, Antonin
No. 249 Konevova
Praha 3(CS)
Inventor: Krepelka, Jiri
No. 26 Madridska
Praha 10(CS)
Inventor: Rezábek, Karel
No. 1551 Pujmanové
Praha 4(CS)
Inventor: Fruhaufová, Maria
No. 97 Vinohradská
Praha 3(CS)
Inventor: Pesak, Milan
No 3158 Poljanovova
Praha 4(CS)
Inventor: Sevcik, Bohumil
No 79 Pohori-Chotoun
Jilove u Prahy(CS)
Inventor: Kral, Josef
No 55 Pohori-Chotoun
Jilove u Prahy(CS)
Inventor: Borovicka, Antonin
Davle I
Davle I(CS)
Inventor: Bilek, Petr
No 14 Kyjevska
Praha 6(CS)
Inventor: Picmausova, Dagmar
No 112 Belehradska
Praha 2(CS)
Inventor: Stuchlik, Josef
No 121 Hlavni
Hrabyne(CS)
Inventor: Picha, Josef
o 469 Za skolou
Jilove u Prahly(CS)
Inventor: Strakova, Jana
No 1116 Nad Lesnim divadlem
Praha 4(CS)

(74) Representative: Wotherspoon, Graham et al
FITZPATRICKS 4 West Regent Street

EP 0 208 417 A2

Glasgow G2 1RS Scotland(GB)

(54) **Use of 1-(8-alpha-ergolinyl)-3,3-diethyl urea derivatives in the treatment of endometritis.**

(57) A composition for the treatment of endometritis in mammalian females which comprises as the physiologically active component a 1-(8alpha-ergolinyl)-3,3-diethylurea of the general formula I

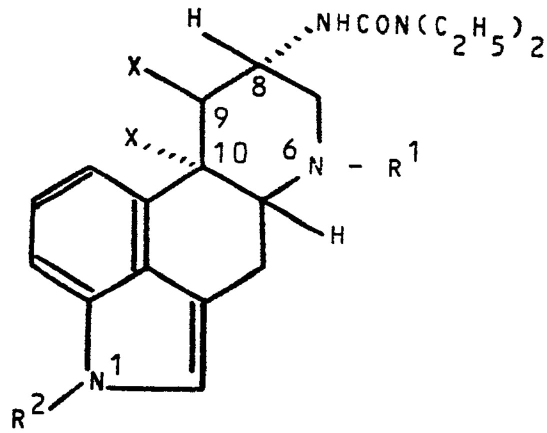

(I)

in which R' represents an alkyl group containing from 1 to 3 carbon atoms, R² represents a hydrogen atom or an alkyl group containing from 1 to 3 carbon atoms and either X stands for a hydrogen atom or both of them form jointly an additional bond between carbon atoms in positions 9 and 10, or a pharmaceutically acceptable acid addition salt thereof. The subject composition of the invention is suitable for treating acute puerperal inflammations and chronic endometritis in mammalian females, especially farm animal females, e.g. cows.

Xerox Copy Centre

## A COMPOSITION FOR THE TREATMENT OF ENDOMETRITIS IN MAMMALIAN FEMALES

The invention relates to a composition for the treatment of endometritis in mammalian females which comprises a 1-(8alpha-ergolinyl)-3,3-diethylurea derivative of the general formula I

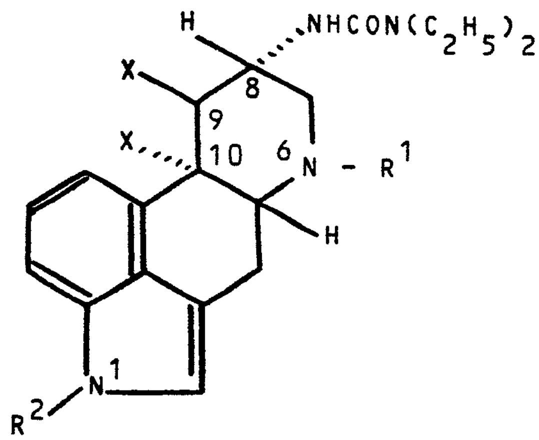

(I)

in which $R^1$ represents an alkyl group containing 1 to 3 carbon atoms, $R^2$ represents a hydrogen atom or an alkyl group containing 1 to 3 carbon atoms and either X stands for a hydrogen atom or both of them together form an additional bond between carbon atoms in positions 9 and 10, or a pharmaceutically acceptable acid addition salt thereof as the physiologically active component, optionally combined with a pharmaceutically acceptable diluent and/or vehicle and/or auxiliary.

Acute puerperal endometrial inflammations and chronic endometrial inflammations (endometritis) are very frequent diseases in females of farm animals, predominantly cows, that affect 10 to 20% of calved animals and result in temporary or in severe cases even permanent sterility. Also in women acute and chronic endometritis are rather common. The contemporary treatment of the disease in farm animals consists of the intrauterine administration of antibiotics, chemotherapeuticals or disinfectants, sometimes supplemented with parenteral uterotonic medication.

According to the present invention ergoline derivatives of the general formula I can be used to advantage in the therapy of endometritis in mammalian females. The compounds of formula I and different methods for their preparation were reported i.a. in Czechoslovak author certificates No. 100 832, 152 153, 156 178 and 217 941, nevertheless the use thereof for the manufacture of compositions for treating endometritis in mammalian females is new and unexpected: generally the application of any ergoline derivatives for the named purpose has never been reported before.

The treatment of acute puerperal and chronic inflammations of endometrium with the suggested compositions, which was developed and verified in females of several mammalian species, is based on the unexpected action of the ergoline derivatives of formula I on the endocrine regulatory system controlling the female genital tract. The administration of these compounds elicits substantial changes in the intrauterine secretion and in the intraluminal content of the uterine cavity, and the endometrial inflammation is suppressed without administration of antibacterial agents.

Model experiments in healthy rats showed that 1-[(5R,8S,10R)-6-methyl-8-ergoline]-3,3-diethylurea (generic name terguride) of formula Ia

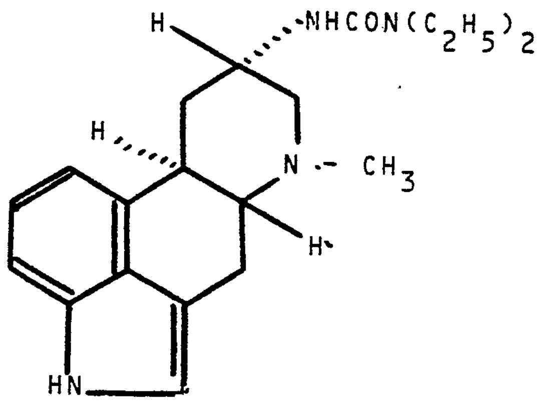

(Ia)

which is a typical example of the aforementioned ergoline derivatives, surprisingly stimulates the action of oestrogenic hormones present in the organism on the intrauterine milieu and vaginal epithelium. The effect of compound Ia on the latter tissue was evidenced microscopically in vaginal smears and involved disappearance of leucocytes, keratinization of epithelial cells, pronounced increase of the lactobacilli count and normalization of intravaginal pH, which is indicative of the non-pathological state of the vagina. The effect of the substance on the intrauterine milieu was shown by higher secretion of the limpid intrauterine fluid, which physiologically enables the penetration of spermatozoa and transport of ova during the ovulation period. All these changes, also including higher content of chlorides in the uterine liquor, deteriorate the conditions for the proliferation of pathogenic microorganisms in the genital tract of mammalian females and facilitate the suppression of bacterial infection.

The effect of compound Ia (terguride) on vaginal cytology and intraluminal uterine secretion was demonstrated by two experiments performed in healthy adult female rats as herein described.

The first experiment was concerned with the effect of short-time administration of compound Ia combined with oestradiol on the quantity of the intraluminal uterine fluid and on the maturation of vaginary epithelial cells, and showed a markedly pronounced influence of compound Ia on the oestrogenic effect. The first group of animals were given 0.05 mg/kg oestradiol valerate (Neofollin Spofa) s.c. on day 1 of the assay. The second group was medicated similarly and received 1 mg/kg of compound Ia p.o. twice daily on days 1 and 2, and the control group (all groups included 6 animals) was untreated. On day 3 the animals were killed and examined. The assay results are summarized in Table I, which demonstrates that the effects of a low dose of oestrogen on the vaginal epithelium and intrauterine fluid were very markedly promoted by the simultaneous administration of compound Ia.

In the other experiment the effects of oestradiol valerate and compound Ia either alone or in combination on the vaginal epithelium and intrauterine fluid were studied for a longer time period at different dosage levels. The experiment was conducted for 20 days. During that time the first group of normal adult female rats (each group consisted of 6 animals) were given 1.25 mg/kg of oestradiol valerate (Neofollin Spofa) s.c. twice weekly at intervals of 3 and 4 days. The second group received 5 mg/kg of compound Ia p.o. once daily. The other two groups were given oestradiol valerate as above and besides 0.5 or 5 mg/kg of compound Ia p.o. The control group was untreated. On day 21 the animals were killed and examined. The results are summarized in Table II, which demonstrates that the pure oestrogenic effect of oestradiol on sexual organs of the females - undisturbed by the action of progesterone, the secretion of which is simultaneously augmented by the administration of an oestrogen -was only achieved when oestradiol valerate was combined with compound Ia. The latter substance, when administered alone, significantly promoted the oestrogenic action of endogenous oestrogens.

The presently reported effect of compound Ia on the intrauterine milieu of females of experimental laboratory animals was confirmed in farm animal females, and it is concluded that the subject compounds of the general formala I have a distinct potential for the treatment of endometritis in farm animals. Thus, the mode of use of the subject composition for the treatment of endometritis in cows, including the respective dosage schedule and obtained results, is illustrated by the following example.

One hundred and eighteen cows with endometritis either untreated before or subsisting after previous local antibiotic treatment were administered a composition containing 20.0 mg of terguride (compound Ia) dihydrogen citrate in 5.0 ml of isotonic saline solution. The pathological condition of the uterus of the treated animals was characterized as puerperal catarrhal to necrotic inflammation with an atonic enlarged uterus charged

with secretions or as chronic inflammation with a copious mucopurulent to purulent discharge; in 19 cows pyometra was ascertained. Staphylococcus pyogenes, Escherichia coli, Proteus vulgaris and Streptococcus viridanswere detected in uterine cervical smears. The composition was injected into the gluteal muscle, and the animals were examined for subsequent 6 days. Pathological uterine symptoms completely disappeared in 64 animals (54.2% of the treated group) and were markedly moderated in 18 (15.3%) whereas in 36 animals (30.5%) the disease was substantially unchanged. In tha animals with subsisting endometritic symptoms the same treatment was repeated once, and the examination was continued for further 5 days. After the single or double administration of the subject composition the endometritic symptoms disappeared completely in 83 cows (70.3%), pronounced amelioration occurred in 16 animals (13.5%), and only in 19 animals (16.2%) the disease remained unchanged. The reported results were achieved within 10 or 11 days of the experimental treatment.

The main advantage of the composition according to the present invention as compared with the known means and methods of veterinary antiendometritic therapy hitherto in use is an important reduction of the required time period from the usual treatment time of 15 to 30 days (intrauterine antibiotic therapy) to a mere 5 to 10 days with obtaining quite comparable therapeutical results. An additional advantage of the subject composi tion consists in its easy, single or double parenteral administration without the fixation of treated animals, whereas the usual intrauterine antibiotic medication requires assistance by auxiliary personnel and must be repeated several times.

The composition of the present invention contains the physiologically active component either in the form of its base or as its water-soluble addition salt with a pharmaceutically acceptable organic or inorganic acid. As compounds of the general formula I, the following typical examples can advantageously be employed:

1-[(5R,8S,10R)-6-methyl-8-ergolinyl]-3,3-diethylurea (compound Ia, generic name terguride),

1-[(5R,8S)-6-methyl-9,10-didehydro-8-ergolinyl]-3,3-diethylurea (compound of formula I, in which $R^1$ represents a methyl group, $R^2$ is a hydrogen atom and the two X together form an additional bond between the carbon atoms in positions 9 and 10, generic name lisuride),

1-[(5R,8S,10R)-6-propyl-8-ergolinyl]-3,3-diethylurea (compound of formula I, in which $R^1$ represents a propyl group and both $R^2$ and the two X stand for hydrogen atoms, generic name proterguride), and

1-[(5R,8S,10R)-1-methyl-6-propyl-8-ergolinyl]-3,3-diethylurea (compound of formula I, in which $R^1$ represents a propyl group, $R^2$ represents a methyl group and the two X symbols stand for hydrogen atoms).

Suitable inorganic acids for the preparation of water-soluble addition salts of the compounds of the general formula I are, e.g. hydrochloric, hydrobromic and phosphoric acid. Suitable organic acids for the same purpose are e.g. acetic, maleic, tartaric, citric, terebic and methanesulphonic acid.

The compositions of the invention can be administered to mammalian females by differenct routes, for example, orally, parenterally or transdermally.

The oral administration of the subject compositions occurs advantageously in the form of capsules, tablets, pellets, granulations, powders, solutions or premixes. The listed dosage forms are manufactured principally by known methods in accordance with procedures of the routine pharmaceutical practice with the use of common solvents, diluents, vehicles and/or auxiliaries such as, e.g. talcum, starches, sugars, cellulose derivatives, taste corrigents (flavours) and stabilizers.

Parenteral administration of these compositions can be effected in the form of subcutaneous, intramuscular or intravenous injections. For the preparation of appropriate injection solutions the active compound of formula I, preferably in the form of an addition salt with a pharmaceutically acceptable organic or inorganic acid, is dissolved in a convenient inert solvent, e.g. distilled water, an isotonic sodium chloride solution, an aqueous glucose solution or the like, if required with the addition of convenient stabilizers and/or solubilizers, and the so obtained injection solution is sterilized by filtration with the use of proper microporous inert materials, filled into ampoules and freeze-dried. Alternatively, said parenteral application of the compounds of formula I can also occur by injecting their oily solutions or suspensions.

The transdermal administration of the subject compositions is possible, e.g. in the form of ointments, creams or solutions. These transdermal dosage forms can be prepared by per se known procedures with the use of a compound of formula I or an addition salt thereof with a pharmaceutically acceptable acid as the active component and one or more common pharmaceutically auxiliaries.

The appropriate dosage of the compositions of the present invention is dependent on the specific choice of a particular compound from among those of the general formula I, further on the administration route to be used, on the nature and gravity of the disease and on the respective mammalian species: starting with the foregoing considerations the dosage is precised by a medical or veterinary

practitioner. Thus, as a rule, cows with endometritis are usually treated with a single intramuscular injection containing 20 mg of the dihydrogen-citrate salt of the aforementioned compound Ia, i.e. approximately 0.05 mg of this salt i.m. per kilogram of weight and, in case of need, the treatment is repeated at the same dosage level after 2 to 10 days.

The composition and formulation procedure of the respective dosage forms of the subject compositions of the present invention is illustrated by the following non-limitative examples.

Example 1

Parenteral injections (liquid)

1-[(5R,8S,10R)-6-methyl-8-ergolinyl]-3,3-diethylurea

| | | |
|---|---|---|
| (compound Ia) dihydrogen-citrate | | 2.0 g |
| sodium chloride | | 4.2 g |
| distilled water | ad | 500.0 ml |

The dihydrogen-citrate is dissolved at room temperature with stirring in an aqueous solution of sodium chloride and the mixture is adjusted by addition of distilled water to the prescribed volume. The obtained solution is sterilized by filtration and filled into 5.0 ml ampoules.

Example 2

Dry injections (freeze-dried)

1-[(5R,8S,10R)-6-methyl-8-ergolinyl]-3,3-diethylurea

| | | |
|---|---|---|
| (compound Ia) dihydrogen-citrate | | 1.2 g |
| citric acid | | 0.3 g |
| sodium hydrogencarbonate | q.s. (ad pH 3.0 to 3.5) | |
| low-molecular-weight dextrane | | 15.0 g |
| distilled water | ad | 300.0 ml |

Citric acid is dissolved in distilled water and the solution is adjusted by gradual addition of sodium hydrogencarbonate to pH 3.0 to 3.5. The hydrogen-citrate and dextrane are dissolved in the obtained medium and the solution is diluted with distilled water to the final volume, sterilized by filtration, filled into 5.0 ml vials and freeze-dried. Immediately before the application the dry solid is dissolved in 5 ml of sterile isotonic aqueous sodium chloride solution.

Example 3

Tablets

1-[(5R,8S,10R)-6-methyl-8-ergolinyl]-3,3-diethylurea

| | |
|---|---|
| (compound Ia) hydrogenmaleate | 0.5 g |
| lactose | 144.5 g |
| calcium hydrogenphosphate dihydrate | 97.0 g |
| maize starch | 180.0 g |
| microcrystalline cellulose | 60.0 g |
| calcium stearate | 18.0 g |

The active component is mixed successively with the above listed auxiliaries, and the mixture is thoroughly homogenized and pressed on a rotary tabletting machine to tablets of 500 mg weight and 13 mm diameter. Each tablet contains 0.5 mg of the active component.

The other compounds of the general formula I can be formulated to appropriate dosage forms similarly.

Table I

| Group Treatment | Intrauterine fluid[a] | Keratinization[c] | Lactobacilli[d] |
|---|---|---|---|
| Zero (control[e]) | 0.04 | 3 (50%) | 1 (17%) |
| Oestradiol valerate[f] | 0.09 | 1 (17%) | 2 (33%) |
| Same + terguride[f] | 0.42 | 6 (100%) | 5 (83%) |

Table II

| Group Treatment | Intrauter. fluid[a] | Leukocytes[b] | Keratinization[c] | Lactobacilli[d] |
|---|---|---|---|---|
| Zero (control[e]) | 0.05 | 3 (50%) | 1 (17%) | 0 |
| Oestradiol valerate[f] | 2.3 | 6 (100%) | 0 | 0 |
| Terguride[f] | 0.4 | 1 (17%) | 5 (83%) | 1 (17%) |
| Oestradiol valerate + terguride[f] 0.5 mg/kg/day | 4.8 | 1 (17%) | 4 (67%) | 5 (83%) |
| Oestradiol valerate + terguride 5 mg/kg/day | 5.4 | 0 | 6 (100%) | 6 (100%) |

Explanations to the preceding Tables:

a -average volume of the intrauterine fluid in millilitres

b, c, d -number of animals with intravaginal massive occurrence (as evaluated microscopically in the smear) of

b -leucocytes (indicating substantial absence of oestrogenic stimulation)

c -predominantly keratinized epithelial cells (a symptom of oestrogenic stimulation)

d -lactobacilli (a criterion of nonpathologic state, i.e. absence of inflammation)

e -positive findings are due to spontaneous cyclic oestrus or proestrus.

f -dosage schedule for Table I and II cf. respectively pp. 2 and 3

## Claims

1. A composition for the treatment of endometritis in mammalian females which comprises a 1-(8alpha-ergolinyl)-3,3-diethylurea derivative of the general formula I

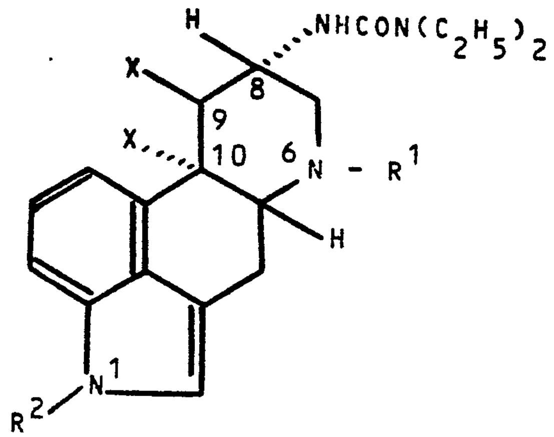

(I)

in which $R^1$ represents an alkyl group containing from 1 to 3 carbon atoms, $R^2$ represents a hydrogen atom or an alkyl group containing from 1 to 3 carbon atoms and either X stands for a hydrogen atom or both of them form jointly an additional bond between carbon atoms in positions 9 and 10, or a pharmaceutically acceptable acid addition salt thereof as the physiologically active component, optionally combined with a pharmaceutically acceptable diluent and/or vehicle and/or auxiliary.

2. A composition according to claim 1 comprising as said active component 1-[(5R,8S,10R)-6-methyl-8-ergolinyl)-3,3-diethylurea of formula Ia or a pharmaceutically acceptable acid addition salt thereof.

3. A 1-(8alpha-ergolinyl)-3,3-diethylurea derivative of the general formula I

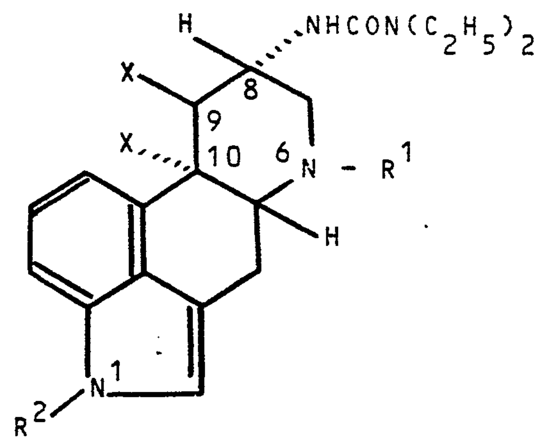

(I)

in which $R^1$ represents an alkyl group containing from 1 to 3 carbon atoms, $R^2$ represents a hydrogen atom or an alkyl group containing from 1 to 3 carbon atoms and either X stands for a hydrogen atom or both of them form jointly an additional bond between carbon atoms in positions 9 and 10, or a pharmaceutically acceptable acid addition salt thereof for use in the treatment of endometritis in mammalian females.

4. 1-[(5R,8S,10R)-6-methyl-8-ergolinyl]-3,3-diethylurea of formula Ia

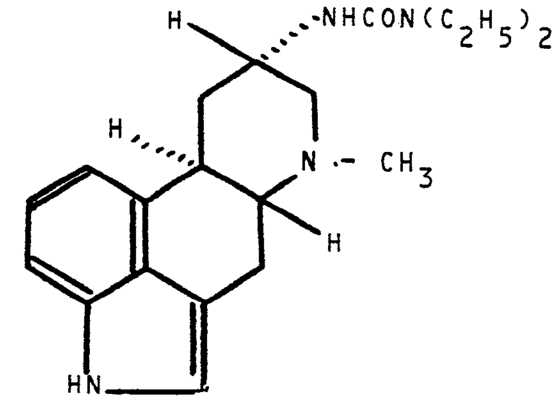

(Ia)

or a pharmaceutically acceptable acid addition salt thereof for use in the treatment of endometritis in mammalian females.

5. The use of a 1-(8alpha-ergolinyl)-3,3-diethylurea derivative of the general formula I

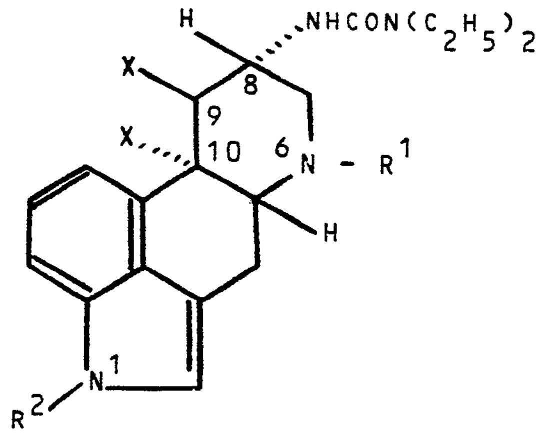

(I)

in which $R^1$ represents an alkyl group containing from 1 to 3 carbon atoms, $R^2$ represents a hydrogen atom or an alkyl group containing from 1 to 3 carbon atoms and either X stands for a hydrogen atom or both of them form jointly an additional bond between carbon atoms in positions 9 and 10, or a pharmaceutically acceptable acid addition salt thereof for the manufacture of a medicament for the treatment of endometritis in mammalian females.

6. The use of 1-[(5R,8S,10R)-6-methyl-8-ergolinyl]-3,3-diethylurea of formula Ia

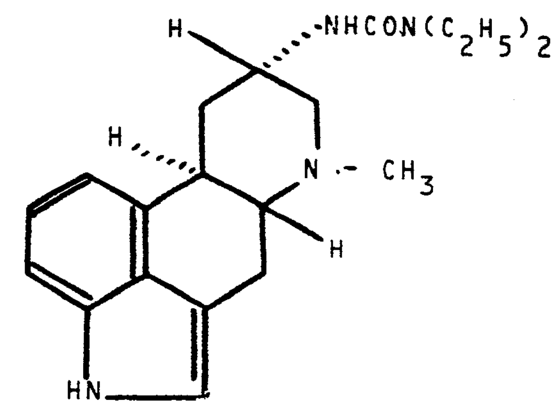

(Ia)

or a pharmaceutically acceptable acid addition salt thereof for the manufacture of a medicament for the treatment of endometritis in mammalian females.